# EUROPEAN PATENT APPLICATION

(11) **EP 4 847 140 A1**
(43) Date of publication of application: **02.12.2026**
(21) Application number: 25744849.8
(22) Date of filing: 20.01.2025
(51) Int. Cl.: B05B 7/08, A45D 34/02, B05B 7/10

(54) **DIFFUSER**

(30) Priority: 23.01.2024 JP 2024007961
(71) Applicant: Revorn Co., Ltd., Tokyo 104-0033 (JP)
(72) Inventor: MATSUOKA, Hiroaki, Tokyo 104-0033 (JP)
(74) Representative: Winter, Brandl - Partnerschaft mbB
(86) International application number: PCT/JP2025/001490
(87) International publication number: WO 2025/159035

(57) **Abstract**

[Problem] To provide a diffuser with which contamination of a fragrance can be suppressed.

[Solution] According to one aspect of the present invention, provided is a diffuser comprising: a plurality of fragrance accommodation units configured so as to accommodate fragrances and emit the fragrances into a mixing space, which is an open space into which the fragrances can be diffused; and an air blowing unit configured so as to feed air toward the mixing space.

## Description

### Technical Field

The present disclosure relates to a diffuser.

### Background Art

As disclosed in PTL 1, diffusers that mix and spray a plurality of fragrances are known.

### Citation List

### Patent Literature

[PTL 1] JP 2021-192906 A

### Summary of Invention

### Technical Problem

In the above-described diffusers, a plurality of oils (fragrances) are mixed in a mixing chamber, and therefore the fragrances adhere to the inner surface of the mixing chamber. As a result, when the fragrance to be used is changed, there is a risk of contamination between the previously used fragrance and the newly used fragrance.

In view of the above circumstances, the present disclosure provides a diffuser capable of suppressing contamination of fragrances.

### Solution to Problem

According to one aspect of the present disclosure, there is provided a diffuser including: a plurality of fragrance storage parts each configured to store a fragrance and release the fragrance into a mixing space that is an open space in which the fragrances are diffused; and an air blowing part configured to feed air toward the mixing space.

According to such an aspect, the fragrances are mixed in the open space outside the diffuser, thereby suppressing adhesion of the fragrances to the inside of the diffuser. Thus, it is possible to suppress contamination between the previously used fragrance and the newly used fragrance.

### Brief Description of Drawings

[FIG. 1] FIG. 1 is a schematic front view of a diffuser 1 according to the present embodiment.
[FIG. 2] FIG. 2 is a schematic rear view of the diffuser 1 according to the present embodiment.
[FIG. 3] FIG. 3 is a schematic plan view of the diffuser 1 according to the present embodiment.
[FIG. 4] FIG. 4 is a schematic bottom view of the diffuser 1 according to the present embodiment.
[FIG. 5] FIG. 5 is a schematic cross-sectional view of the diffuser 1 according to the present embodiment, taken along a cross section parallel to the vertical direction.
[FIG. 6] FIG. 6 is a schematic plan view for explaining the arrangement of fragrance storage parts 3 in the diffuser 1.
[FIG. 7] FIG. 7 is a part of a schematic cross-sectional view taken along line V-V of FIG. 6.
[FIG. 8] FIG. 8 is a partially enlarged view of the vanes in the cross-sectional view of FIG. 5.
[FIG. 9] FIG. 9 is a block diagram showing the hardware configuration of a controller 10.
[FIG. 10] FIG. 10 is a graph showing an example of adjustment of the fragrance release amount by the controller 10.
[FIG. 11] FIG. 11 is a graph showing another example of adjustment of the fragrance release amount by the controller 10.
[FIG. 12] FIG. 12 is a graph showing still another example of adjustment of the fragrance release amount by the controller 10.

### Description of Embodiments

Hereinafter, embodiments of the present disclosure will be described with reference to the drawings. Various features shown in the embodiments described below can be combined with each other.

### <Diffuser>

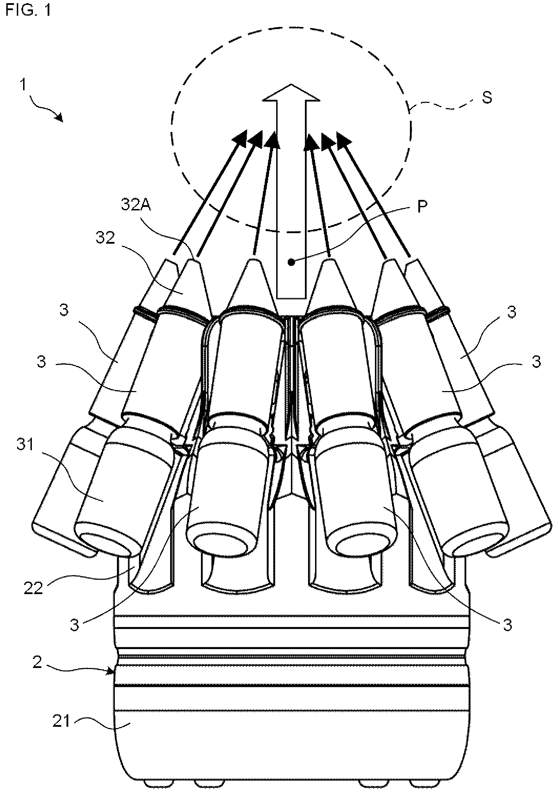

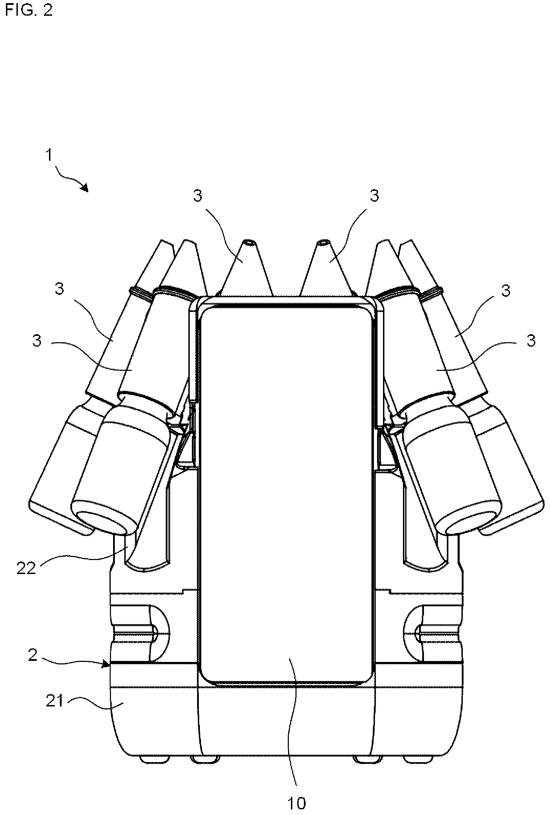

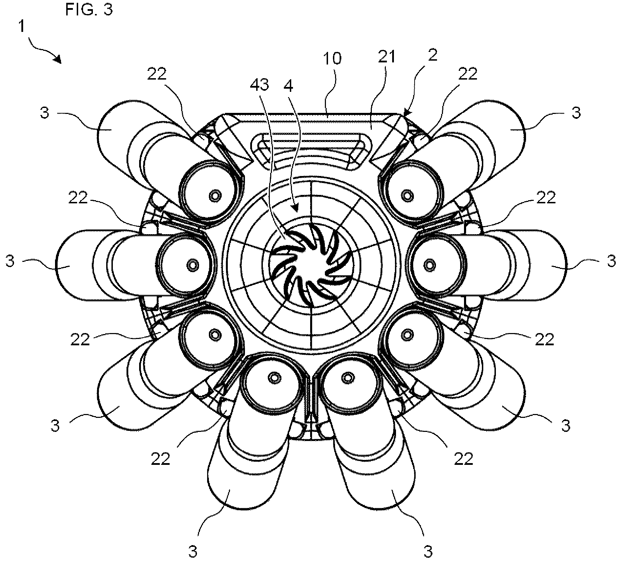

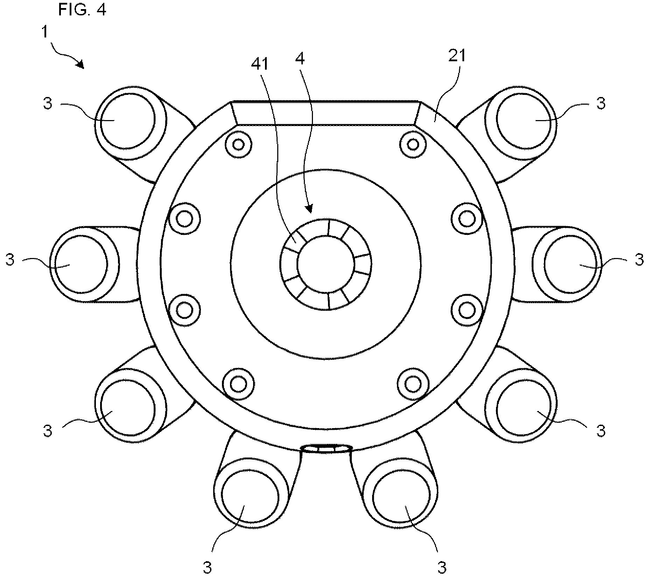

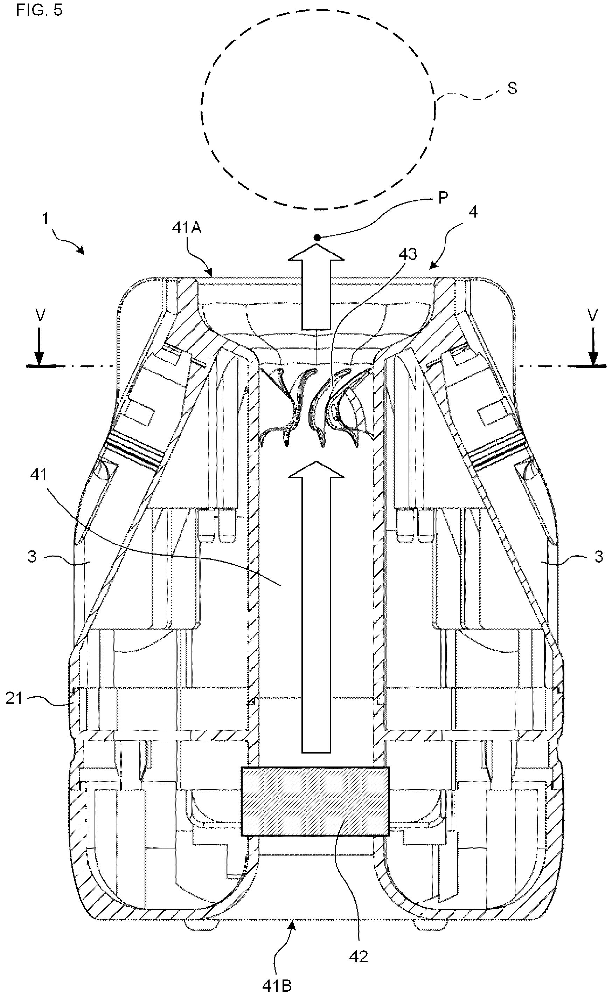

FIG. 1 is a schematic front view of a diffuser 1 according to the present embodiment. FIG. 2 is a schematic rear view of the diffuser 1 according to the present embodiment. FIG. 3 is a schematic plan view of the diffuser 1 according to the present embodiment. FIG. 4 is a schematic bottom view of the diffuser 1 according to the present embodiment. FIG. 5 is a schematic cross-sectional view of the diffuser 1 according to the present embodiment, taken along a cross section parallel to the vertical direction.

The diffuser 1 is configured to supply air in which a plurality of types of fragrances are diffused and mixed. As shown in FIGS. 1 to 5, the diffuser 1 includes a housing 2, a plurality of fragrance storage parts 3, an air blowing part 4, and a controller 10.

### <Housing 2>

The housing 2 shown in FIG. 1 is a cylindrical member inside which the air blowing part 4 described later is arranged. The housing 2 includes a main body 21 and a plurality of bases 22.

As shown in FIG. 1, the main body 21 is a tubular body having a central axis that is parallel to the vertical direction (that is, extends in the up-down direction) in a state where the diffuser 1 is used. An air passage 41 (see FIG. 5) of the air blowing part 4 described later is arranged inside the main body 21 (that is, in the hollow portion). Air is discharged upward from the upper end of the main body 21 by the air blowing part 4. In addition, a battery, electric circuits, a drive source (motor), and the like are housed inside the main body 21.

As shown in FIG. 3, the plurality of bases 22 are provided on the outer peripheral surface of the housing 2. In addition, the plurality of fragrance storage parts 3 described later are attached respectively to the plurality of bases 22. According to such an aspect, the diffuser 1 can be made compact, and the fragrance storage parts 3 can be easily replaced.

Each of the plurality of bases 22 is a holder configured to detachably hold the fragrance storage part 3. The plurality of bases 22 are arranged on the outer peripheral surface of the main body 21 so as to be aligned in the circumferential direction of the main body 21. Specifically, each base 22 is formed by a portion in which the outer peripheral surface (that is, a tapered surface) of a reduced-diameter portion of the main body 21 is recessed radially inward. The reduced-diameter portion of the main body 21 is provided at the upper end portion of the main body 21 and is a portion that is reduced in diameter toward the upper end (the air outlet).

Each base 22 has, for example, an engaged portion (for example, a recess with which a claw engages) with which an engaging portion (for example, a protrusion such as a claw) provided on the fragrance storage part 3 can engage. In addition, each base 22 has a terminal for supplying electric power (voltage) for atomizing the fragrance to the fragrance storage part 3.

### <Fragrance storage part 3>

The plurality of fragrance storage parts 3 are each configured to store a fragrance. In addition, as shown in FIG. 1, the plurality of fragrance storage parts 3 are each configured to release the fragrance into a mixing space S that is an open space in which the fragrances can be diffused.

Each fragrance storage part 3 includes a reservoir 31 and a nozzle 32. The reservoir 31 is a cylindrical member that stores a liquid fragrance. Different kinds of fragrances are stored in the reservoirs 31 of the plurality of fragrance storage parts 3, respectively.

Each nozzle 32 is configured to atomize the fragrance in the reservoir 31 and release the fragrance upward (toward the mixing space S). The nozzle 32 atomizes the fragrance by, for example, electric power supplied from the housing 2. Known methods may be used as the atomization method, and examples thereof include excitation by high-frequency (ultrasonic) vibration.

Each fragrance storage part 3 is attached to the housing 2 in an orientation in which the discharge opening 32A of the nozzle 32 faces upward. In addition, the heights of the discharge openings 32A of the nozzles 32 of the plurality of fragrance storage parts 3 (that is, the positions of the discharge openings 32A in the axial direction of the housing 2) are the same. The release direction of the fragrance from each nozzle 32 intersects with the vertical direction (that is, is inclined relative to the vertical direction).

Each fragrance storage part 3 is a replaceable cartridge containing a desired fragrance selected by a user and attached to the housing 2. When the fragrance in the reservoir 31 has been consumed, the fragrance is replenished by the user replacing the fragrance storage part 3. Each fragrance storage part 3 may be configured such that the entire fragrance storage part 3 is replaceable, or may be configured such that only the reservoir 31 is replaced.

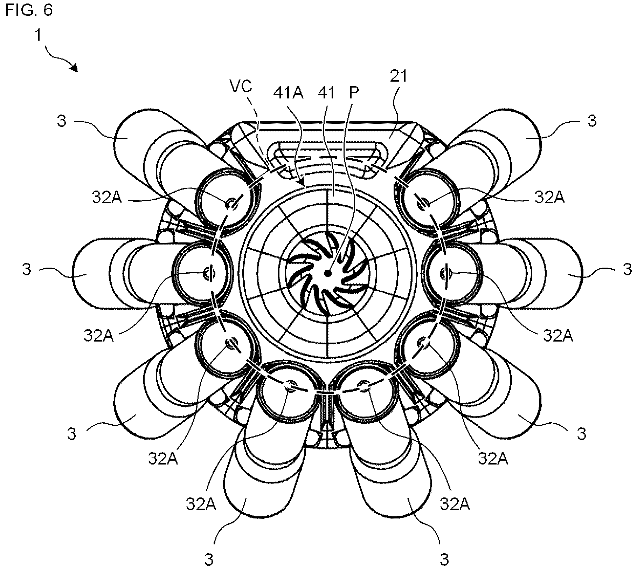

FIG. 6 is a schematic plan view for explaining the arrangement of the fragrance storage parts 3 in the diffuser 1. As shown in FIG. 6, the diffuser 1 may include, as the plurality of fragrance storage parts 3, three or more fragrance storage parts 3 whose discharge openings 32A for the fragrances are arranged on a single imaginary circle VC. Here, the center P of the imaginary circle VC is a point on the central axis of the air passage 41 of the air blowing part 4 described later (that is, a point on the central axis of the main body 21), and in a plan view, coincides with the center point of a circle formed by a cross section perpendicular to the axial direction of the air passage 41. According to such an aspect, it is possible to suppress non-uniformity in mixing of the fragrances respectively released from the plurality of fragrance storage parts 3. In addition, the plurality of fragrance storage parts 3 are arranged so as to surround the air passage 41 from the radially outer side.

Note that, in the example of FIG. 6, the diffuser 1 includes eight fragrance storage parts 3; however, the number of the fragrance storage parts 3 is not limited thereto and may be, for example, 100 or more.

### <Air Blowing Part 4>

As shown in FIG. 5, the air blowing part 4 is configured to feed air toward the mixing space S. Specifically, the air blowing part 4 is configured to feed air to the mixing space S from below. According to such an aspect, the diffusion effect of the fragrances can be enhanced. The air blowing part 4 includes an air passage 41, a fan 42, and vanes 43.

The air passage 41 is formed of a cylindrical straight tube. The air passage 41 includes an outlet 41A through which air is discharged and an inlet 41B through which air is introduced into the air passage 41. The outlet 41A is arranged above the inlet 41B. In addition, the air passage 41 expands in diameter toward the outlet 41A in the direction of air flow. The diameter of the outlet 41A is larger than the diameter of the other portions of the air passage 41. In addition, as shown in FIG. 6, the diameter of the outlet 41A is smaller than the diameter of the imaginary circle VC.

The fan 42 shown in FIG. 5 is configured to feed air toward the mixing space S. The fan 42 is arranged inside the air passage 41. The fan 42 draws air from the inlet 41B of the air passage 41 and feeds the air toward the outlet 41A of the air passage 41. As the fan 42, for example, a motor fan, a piezo fan using a piezoelectric element, or the like can be used.

The air blowing part 4 is configured to feed air toward the center P of the imaginary circle VC using the air passage 41 and the fan 42. This suppresses the fragrance released from each fragrance storage part 3 from entering and adhering to the air blowing part 4 (the inside of the air passage 41).

The plurality of vanes 43 are arranged between the fan 42 and the mixing space S, and are arranged so as to impart rotation to the air discharged by the fan 42. According to such an aspect, a vortex is generated in the air discharged from the fan 42, and thus, the mixing effect of the fragrances can be enhanced.

The plurality of vanes 43 are arranged on the downstream side (lower side) of the outlet 41A in the air passage 41. Specifically, the plurality of vanes 43 are arranged in a region closer to the outlet 41A than to the fan 42 inside the air passage 41.

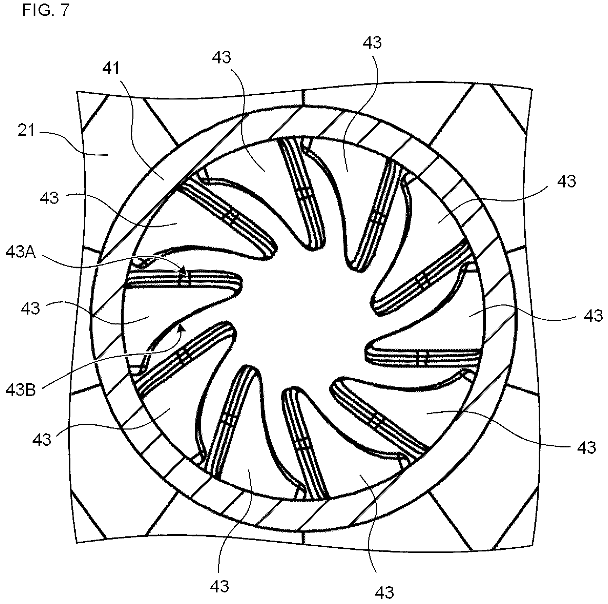

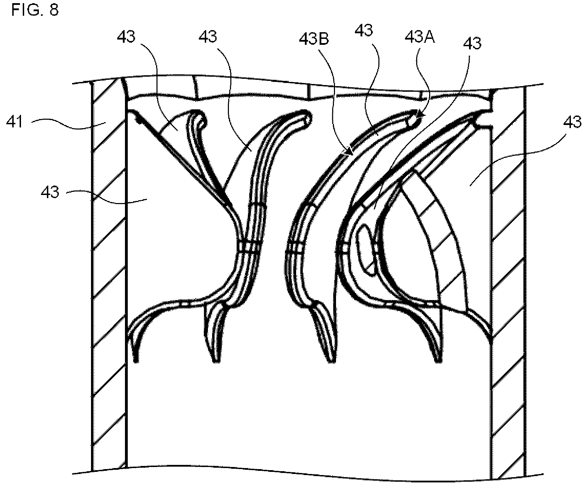

FIG. 7 is a part of a schematic cross-sectional view taken along line V-V of FIG. 6. FIG. 8 is a partially enlarged view of the vanes in the cross-sectional view of FIG. 5. As shown in FIG. 7, each of the plurality of vanes 43 protrudes radially inward from the inner peripheral surface of the air passage 41. The plurality of vanes 43 all have the same shape and are arranged at equal intervals along the circumferential direction of the air passage 41.

Each vane 43 has a shape configured to impart a flow in the circumferential direction of the air passage 41 to air fed from below when the air collides with the vane 43. Specifically, each vane 43 includes a first edge 43A and a second edge 43B. The first edge 43A extends from the inner peripheral surface of the air passage 41 toward the center of the air passage 41. In addition, as shown in FIG. 8, the first edge 43A constitutes an upper end of each vane 43. As shown in FIG. 7, the second edge 43B extends from an end portion of the first edge 43A in the counterclockwise direction while curving so as to be concave toward the radially outer side of the air passage 41 in a plan view (as viewed from above). Furthermore, as shown in FIG. 8, the second edge 43B extends downward from the end portion of the first edge 43A while curving so as to be convex toward the radially inner side of the air passage 41.

When the air discharged from the fan 42 collides with the vanes 43, a clockwise flow is generated in the air in a plan view. As a result, the air is supplied to the mixing space S while rotating clockwise. The atomized fragrances respectively released from the plurality of fragrance storage parts 3 are mixed and diffused by the rotating air. The direction of rotation of the air caused by the vanes 43 may be counterclockwise in a plan view.

### <Controller 10>

The controller 10 shown in FIG. 2 is configured to execute activation of the diffuser 1, reception of inputs of operating conditions and the like, control of the amount of fragrance released from the fragrance storage parts 3 (that is, the mixing ratio of the plurality of fragrances), and the like.

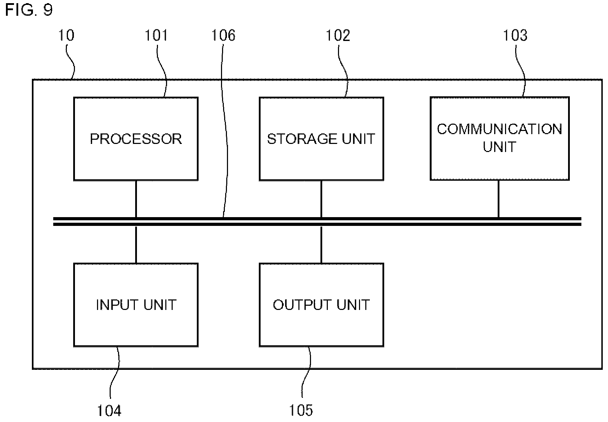

FIG. 9 is a block diagram showing the hardware configuration of the controller 10. The controller 10 includes a processor 101, a storage unit 102, a communication unit 103, an input unit 104, an output unit 105, and a communication bus 106. The processor 101, the storage unit 102, the communication unit 103, the input unit 104, and the output unit 105 are electrically connected via the communication bus 106 inside the controller 10.

The processor 101 processes and controls the overall operation related to the controller 10. The processor 101 is, for example, a central processing unit (CPU). The processor 101 implements various functions executed by the controller 10 by reading a predetermined program stored in the storage unit 102. In other words, information processing performed by software stored in the storage unit 102 may be specifically implemented by the processor 101, which is an example of hardware, and thereby executed as each functional unit included in the processor 101. The processor 101 is not limited to a single processor, and the controller 10 may include a plurality of processors 101 for respective functions. Moreover, the controller 10 may have a configuration combining these.

The storage unit 102 stores various kinds of information defined by the aforementioned description. The storage unit 102 may be implemented, for example, as a storage device such as a solid state drive (SSD) that stores various programs and the like executed by the processor 101, or as a memory such as a random access memory (RAM) storing temporarily necessary information (arguments, arrays, or the like) related to program operations. The storage unit 102 stores various programs, variables, and the like executed by the processor 101.

It is preferable for the communication unit 103 to include wired communication means such as USB, IEEE 1394, Thunderbolt (registered trademark), and wired LAN network communication. The communication unit 103 may also include wireless LAN network communication, mobile communication such as LTE/5G, and BLUETOOTH (registered trademark) communication as necessary. That is, it is more preferable to implement the communication unit 103 as a set of these multiple types of communication means. In other words, the controller 10 may communicate various types of information from the outside via the communication unit 103 and a network.

The input unit 104 receives an operation input performed by a user. The operation input is transferred to the processor 101 as an instruction signal via the communication bus 106. The processor 101 may execute predetermined control or computation based on the transferred instruction signal as needed. The input unit 104 may be included in the housing of the controller 10 or may be externally attached. For example, the input unit 104 may be implemented as a touchscreen integrated with the output unit 105. When the input unit 104 is implemented as a touchscreen, the user can input operations such as tapping, swiping, and the like on the input unit 104.

The output unit 105 displays a screen of a graphical user interface (GUI) that can be operated by a user. The output unit 105 may be included in the housing of the controller 10 or may be externally attached. Specifically, the output unit 105 may be implemented as a display device such as a CRT display, a liquid crystal display, an organic EL display, a plasma display, or the like.

The controller 10 may perform activation, setting, and the like of the diffuser 1 in response to an input from the input unit 104, or may perform activation, setting, and the like of the diffuser 1 in response to an input from an information processing terminal, a remote controller, or the like owned by the user.

The controller 10 (processor 101) executes an acquisition step of acquiring adjustment information that specifies a mixing ratio of the fragrances respectively stored in the plurality of fragrance storage parts 3, and an adjustment step of adjusting the amount of fragrance released from each of the plurality of fragrance storage parts 3 based on the adjustment information.

In the acquisition step, the adjustment information (mixing ratio) may be acquired by an input from the user, or the adjustment information may be acquired from a server. The input from the user may be, for example, received not only as a direct input to the controller 10 but also as adjustment information input on an information processing terminal owned by the user and received from the information processing terminal.

Furthermore, the adjustment information acquired from the server may be generated by an adjustment information creation model implemented by artificial intelligence. The adjustment information creation model is a trained model that has been trained, for example, so as to be capable of receiving impression information such as text, images, and audio as an input and outputting adjustment information for generating a scent corresponding to the impression information. The adjustment information creation model is constructed by machine learning using, for example, training data in which impression information is associated with a mixing ratio (recipe) of fragrances corresponding to the impression information. For example, the server inputs impression information received from the information processing terminal of the user into the adjustment information creation model, causes the adjustment information creation model to output the adjustment information, and transmits the adjustment information to the controller 10.

In the adjustment step, based on the acquired adjustment information, fragrance is sprayed in a predetermined amount from at least one fragrance storage part 3 (specifically, the fragrance storage part 3 that stores the fragrance specified by the adjustment information). The spraying of the fragrance from the fragrance storage part 3 may be performed continuously, or may be performed intermittently within a range where a desired scent can be generated.

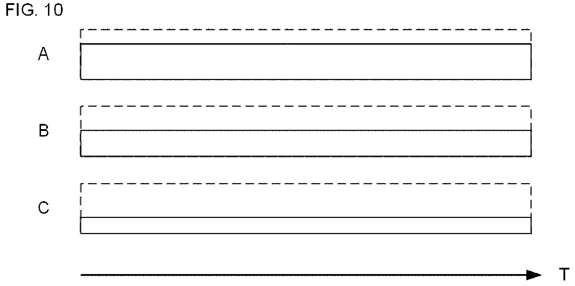

FIG. 10 is a graph showing an example of adjustment of the fragrance release amount by the controller 10. FIG. 10 shows, in a time-series manner, the magnitudes of the voltages applied to the respective fragrance storage parts 3 in the case where three kinds of fragrances, namely fragrance A, fragrance B, and fragrance C, are mixed at a mixing ratio of 8:5:2. Each fragrance storage part 3 releases the fragrance in an amount proportional to the applied voltage value. In FIG. 10, the dashed line for each fragrance indicates the maximum value of the voltage in the fragrance storage part 3 (that is, the voltage value when the fragrance is released in the maximum amount), and the solid line indicates the magnitude of the applied voltage. In addition, the horizontal axis in FIG. 10 represents time T. In the example of FIG. 10, a voltage of 80% of the maximum value is continuously applied to the fragrance storage part 3 for fragrance A, a voltage of 50% of the maximum value is continuously applied to the fragrance storage part 3 for fragrance B, and a voltage of 20% of the maximum value is continuously applied to the fragrance storage part 3 for fragrance C. As a result, a scent in which fragrance A, fragrance B, and fragrance C are mixed at a mixing ratio of 8:5:2 is generated in the diffuser 1.

In this manner, by changing the voltage for each fragrance storage part 3 and continuously applying the voltage (continuing the spraying by the fragrance storage part 3), it is possible to suppress the occurrence of operating noise caused by switching spraying on and off.

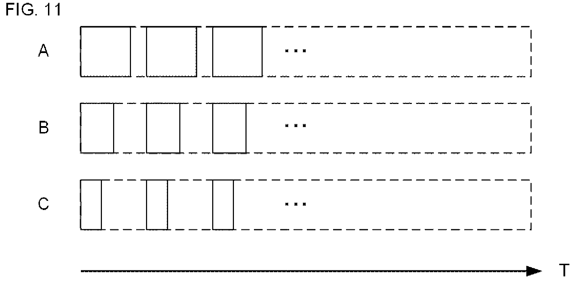

FIG. 11 is a graph showing another example of adjustment of the fragrance release amount by the controller 10. FIG. 11 shows, similarly to FIG. 10, the magnitudes of the voltages applied to the respective fragrance storage parts 3 in a time-series manner in the case where three kinds of fragrances, namely fragrance A, fragrance B, and fragrance C, are mixed at a mixing ratio of 8:5:2. In the example of FIG. 11, while the voltages applied to the respective fragrance storage parts 3 are the same, the times during which the voltages are applied are set at a ratio of 8:5:2. Specifically, one cycle is defined as simultaneously applying voltage to the three fragrance storage parts 3, and stopping the application of the voltage to the corresponding fragrance storage part 3 in the order of fragrance C, fragrance B, and fragrance A when respective predetermined periods of time (the set release times) have elapsed, and this cycle is repeated. Thus, a scent in which fragrance A, fragrance B, and fragrance C are mixed at a mixing ratio of 8:5:2 is generated in the diffuser 1. Note that the period of one cycle (a cycle period of voltage application to the three fragrance storage parts 3) is, for example, in a range of 100 ms to 1 s.

In this manner, by changing the application time of the voltage for each fragrance storage part 3 without changing the voltage, a voltage adjustment mechanism is not required. Therefore, for example, a desired scent can be generated by using PWM control.

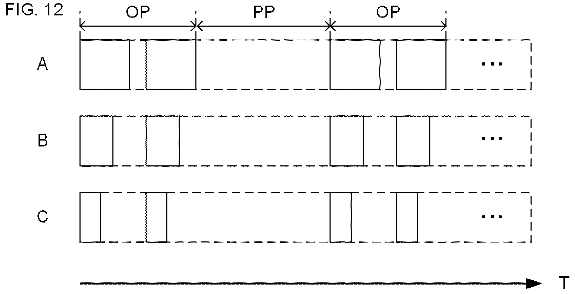

FIG. 12 is a graph showing still another example of adjustment of the fragrance release amount by the controller 10. The control shown in FIG. 12 is obtained by providing, in the control shown in FIG. 11, an interval period PP during which no voltage is applied to any of the fragrance storage parts 3. The interval period PP is longer than the cycle period of voltage application, and is, for example, on the order of several minutes to several tens of minutes. In addition, a period during which the cycle is repeated before the interval period PP (a spray operation period OP) is, for example, on the order of several seconds.

In this manner, by providing the interval period, it is possible to suppress the occurrence of operating noise caused by switching spraying on and off within a certain period while eliminating the need for the voltage adjustment mechanism.

### 4. Effects

The effects of the present embodiment are summarized as follows. That is, the fragrances are mixed in the open space outside the diffuser 1, and therefore adhesion of the fragrances to the inside of the diffuser 1 is suppressed. Thus, it is possible to suppress contamination between the previously used fragrance and the newly used fragrance.

Although the embodiments of the present disclosure have been described above, the present disclosure is not limited thereto and can be modified as appropriate without departing from the technical concept of the present disclosure.

### 5. Others

The diffuser 1 may include a cover that covers at least a part of the plurality of fragrance storage parts 3 (for example, a part other than the discharge opening 32A of each fragrance storage part 3).

The diffuser 1 may include a heater configured to heat at least one of the fragrance storage part 3 itself, the fragrance released from the fragrance storage part 3, the air passage 41 itself, and the air fed by the air blowing part 4. This improves the diffusibility of the fragrance.

The diffuser 1 may include a water reservoir that stores water and a humidifier that atomizes the water in the water reservoir. The water reservoir is arranged, for example, at a lower portion of the housing 2. The water vapor (or mist) generated by the humidifier is sprayed outside the diffuser 1, either alone or mixed with the fragrance, by the air blowing part 4.

The diffuser 1 may be self-propelled. For example, the diffuser 1 may include rolling elements such as wheels or rollers, and a motor that drives the rolling elements. In addition, the diffuser 1 may include a grip for carrying.

The diffuser 1 may include an automatic cleaning function for the air blowing part 4 (at least one of the air passage 41, the fan 42, and the vanes 43). For example, the diffuser 1 may include a brush for cleaning the inside of the air blowing part 4, and a mechanism for driving the brush.

The housing 2 does not necessarily have to be cylindrical as long as it can hold the fragrance storage parts 3 and the air blowing part 4.

The discharge openings 32A for fragrances of the plurality of fragrance storage parts 3 do not necessarily have to be arranged on one imaginary circle as long as the plurality of fragrance storage parts 3 can respectively release fragrances into the mixing space. For example, the plurality of fragrance storage parts 3 may be arranged in a single row in the lateral direction. In addition, the fragrance storage parts 3 do not necessarily have to be attached to the housing 2.

The air blowing part 4 does not necessarily have to include the vanes 43. That is, the air blowing part 4 does not necessarily have to be configured to feed air containing a vortex to the mixing space.

In addition, the air blowing part 4 may be configured to feed air to a plurality of mixing spaces. For example, air may be fed from a single air blowing part 4 to a first mixing space into which fragrances are released from a plurality of fragrance storage parts 3 serving as a plurality of first release mechanisms, and to a second mixing space into which fragrances are released from a plurality of fragrance storage parts 3 serving as second release mechanisms different from the first release parts.

Furthermore, the air blowing part 4 does not necessarily have to be configured to feed air to the mixing space from below. For example, the air blowing part 4 may be configured to feed air to the mixing space from above, or may be configured to feed air to the mixing space from the side. For example, when the air blowing part 4 feeds air to the mixing space from the side, the diffuser 1 can be used while being hung on a wall.

The present disclosure may be provided in each of the following aspects.
(1) A diffuser comprising: a plurality of fragrance storage parts each configured to store a fragrance and release the fragrance into a mixing space that is an open space in which the fragrances are diffused; and an air blowing part configured to feed air toward the mixing space.
   According to such an aspect, the fragrances are mixed in the open space, and therefore adhesion of the fragrances to the inside of the diffuser is suppressed. Thus, it is possible to suppress contamination between the previously used fragrance and the newly used fragrance.
(2) The diffuser according to (1), wherein: the air blowing part is configured to feed air to the mixing space from below.
   According to such an aspect, the diffusion effect of the fragrances can be enhanced.
(3) The diffuser according to (1) or (2), wherein: the plurality of fragrance storage parts include three or more fragrance storage parts whose discharge openings for the fragrances are arranged on a single imaginary circle, and the air blowing part is configured to feed air toward a center of the imaginary circle.
   According to such an aspect, it is possible to suppress non-uniformity in mixing of the fragrances respectively released from the plurality of fragrance storage parts.
(4) The diffuser according to any one of (1) to (3), wherein: the air blowing part includes a fan configured to feed air toward the mixing space, and a plurality of vanes arranged between the fan and the mixing space and arranged so as to impart rotation to the air discharged by the fan.
   According to such an aspect, a vortex is generated in the air discharged from the fan, and thus, the mixing effect of the fragrances can be enhanced.
(5) The diffuser according to any one of (1) to (4), further comprising: a cylindrical housing inside which the air blowing part is arranged, wherein an outer peripheral surface of the housing is provided with a plurality of bases to which the plurality of fragrance storage parts are respectively attached.

According to such an aspect, the diffuser can be made compact, and the fragrance storage parts can be easily replaced.

Of course, the present disclosure is not limited thereto.

Finally, various embodiments of the present disclosure have been described, but these are presented as examples and are not intended to limit the scope of the disclosure. The novel embodiments can be implemented in various other forms, and various omissions, replacements, and modifications can be made within the scope of the gist of the disclosure. The embodiments and their modifications are included in the scope and the gist of the disclosure and are included in the scope of the invention described in the claims and the equivalent scope thereof.

### Reference Signs List

1: Diffuser
2: Housing
3: Fragrance storage part
4: Air blowing part
10: Controller
21: Main body
22: Base
31: Reservoir
32: Nozzle
32A: Discharge opening
41: Air passage
41A: Outlet
41B: Inlet
42: Fan
43: Vane
43A: First edge
43B: Second edge
101: Processor
102: Storage unit
103: Communication unit
104: Input unit
105: Output unit
106: Communication bus
OP: Spray operation period
P: Center
PP: Interval period
S: Mixing space
VC: Imaginary circle

## Claims

1. A diffuser comprising:
a plurality of fragrance storage parts each configured to store a fragrance and release the fragrance into a mixing space that is an open space in which the fragrances are diffused; and
an air blowing part configured to feed air toward the mixing space.

2. The diffuser according to claim 1, wherein:
the air blowing part is configured to feed air to the mixing space from below.

3. The diffuser according to claim 1 or 2, wherein:
the plurality of fragrance storage parts include three or more fragrance storage parts whose discharge openings for the fragrances are arranged on a single imaginary circle, and
the air blowing part is configured to feed air toward a center of the imaginary circle.

4. The diffuser according to any one of claims 1 to 3, wherein:
the air blowing part includes
a fan configured to feed air toward the mixing space, and
a plurality of vanes arranged between the fan and the mixing space and arranged so as to impart rotation to the air discharged by the fan.

5. The diffuser according to any one of claims 1 to 4, further comprising:
a cylindrical housing inside which the air blowing part is arranged, wherein
an outer peripheral surface of the housing is provided with a plurality of bases to which the plurality of fragrance storage parts are respectively attached.
